Europäisches Patentamt

European Patent Office

Office européen des brevets

(19)

(11) Publication number: **0 315 447**
A2

## (12) EUROPEAN PATENT APPLICATION

(21) Application number: 88310354.1

(22) Date of filing: 03.11.88

(51) Int. Cl.⁴: **G 01 N 33/68**
G 01 N 33/543, G 01 N 33/577

(30) Priority: 06.11.87 JP 279362/87

(43) Date of publication of application:
10.05.89 Bulletin 89/19

(84) Designated Contracting States:
AT BE CH DE ES FR GB IT LI NL SE

(71) Applicant: **TEIJIN LIMITED**
11 Minami Honmachi 1-chome Higashi-ku
Osaka-shi Osaka-fu (JP)

(72) Inventor: **Suzuki, Hideaki**
2-7-18, Sakuramachi
Koganei-shi Tokyo (JP)

**Kubota, Takaharu**
211 Teijin Tama Apart 3-18-4, Tamadaira
Hino-shi Tokyo (JP)

**Hasegawa, Ryoichi**
1-28-5-302, Ozu-cho
Iwakuni-shi Yamaquchi (JP)

**Koike, Yukiya**
102 First Manshon 399-1, Miya
hino-shi Tokyo (JP)

(74) Representative: **Arthur, Bryan Edward et al**
Withers & Rogers 4 Dyer's Buildings Holborn
London EC1N 2JT (GB)

(54) Method of immunological measurement of human protein S and reagent and kit therefor.

(57) A method of immunological measurement of human protein S, which comprises performing an immunological measurement of human protein S by using an antibody bound to an insoluble carrier and a labelled antibody, wherein a polyclonal antibody which specifically recognizes human protein S is used as either one of the antibodies, and a monoclonal antibody which does not recognize the complex of C4bp of a human complement system control factor with protein S but can specifically recognize free human protein S to be bound thereto is used as the other antibody.

Fig.1 CALIBRATION CURVE FOR MEASUREMENT
FOR HUMAN PROTEIN S (PS)

Description

# METHOD OF IMMUNOLOGICAL MEASUREMENT OF HUMAN PROTEIN S AND REAGENT AND KIT THEREFOR

## BACKGROUND OF THE INVENTION

### 1. Field of the Invention

The present invention relates to a method of immunological measurement of human protein S in a solution, and a measurement reagent and a kit used therefor. More specifically, it relates to a method of immunological measurement of human protein S which can measure only free human protein S specifically and at a high sensitivity, without measuring the complex of C4bp of a human complement system control factor and protein S, and a measurement reagent and a kit used therefor.

### 2. Description of the Related Art

Protein S is a protein dependent on vitamin K, as in the case of protein C, and was separated from bovines and humans by DiScipio et al in 1977 [see DiScipio, R.G., Hermodson, M.A., Yates, S.G. and Davie, E.W.: Biochemistry, 16, 698 - 706 (1977)]. Human protein S is contained in plasma at a level of about 10 mg/l, and is a single-stranded glycoprotein having a molecular weight of 69,000. The structure thereof is similar to that of other vitamin K-dependent factors, and has about 10 $\gamma$-carboxyglutamic acid (Gla) at the $NH_2$ terminus. Human protein S exists in two forms in blood; one being free protein S, which free protein acts as an aiding factor for activated protein C, and the other is non-covalently bound to the high molecular weight C4b bound protein (C4bp) which is a control factor of the complement system. The ratio of the free protein S and the bound protein S is approximately 1 : 1.

The importance of the function of protein S in the C4bp-protein S complex resides in its very strong affinity to the negatively charged surface of a phospholipid [Nelsestuen, G.L., Kisiel, W. and DiScipio, R.G.: Biochemistry, 17: 2134 - 2138 (1978)]. It is considered that, when a cell is damaged or activated, the Gla-domain of protein S is bound to a phospholipid in the presence of $Ca^{2+}$, and further, C4bp is bound to the protein S to form a complex, to thereby exhibit its function [Dahlback, B.: Semin. Thromb. Haemostas., 10: 139 - 148 (1984)].

Regarding the function concerning coagulation and the fibrinogenolysis system, recent studies have clarified that this plays an extremely important role in the control mechanism thereof, and the physiological significance thereof is attracting attention with respect to the relationship thereof to thrombosis. It has been reported that a congenital deficiency of protein S can be a cause of thrombosis [Comp, P.C., Nixon, R.R., Cooper, H.R. and Esmon, C.T.: J. Clin. Invest., 74: 2082 - 2088 (1984)].

Accordingly, if the mechanism of protein S can be clarified, and further, the antigenic amount and active amount of protein S in blood can be measured to grasp the behavior thereof, this would have a very significant affect in the fields of basic medicine and clinical medicine.

As the method of measuring protein S known in the art, there can be included the Laurel method using an antiserum to protein S, IRMA (i.e., immunoradiometricassay using a polyclonal antibody) and EIA (i.e., enzyme immmonoassay), but the measured values obtained by these methods include both free protein S and a protein S forming a complex with C4bp.

For this reason, in the method of measuring only free protein S in the prior art, it is necessary to remove the complex of C4bp and protein S by precipitation, by treating the test sample with an aqueous polyethylene glycol solution, but this measurement method has a drawback in that the operations are very cumbersome.

On the other hand, a monoclonal antibody has been widely utilized recently for analysis of the function and structure of an antigenic protein or an immunological measurement (ETA, RIA), as it has certain advantages in that it is specific to a single antigenic determinant, and can stably produce antibodies having the same specificity. Particularly, for a functional analysis and molecular analysis of an antigenic protein, it can be a powerful means of determining the site participating in the function of the antigenic protein, or an antibody recognizing a special structural site.

Also, in the method of measurement of free human protein S, the antigen amount of free human protein S at a very high specificity has been measured by using two kinds of monoclonal antibodies which discriminate free protein S from the protein S forming the complex (see Japanese Unexamined Patent Publication No. 63-151856).

Nevertheless, in the method of a measurement of free human protein S by using two kinds of monoclonal antibodies, because of the characteristic of a monoclonal antibody whereby it does not react with sites other than the specific site of an antigen, it sometimes does not have a sufficiently high affinity to an antigen, and therefore, there is a demand for a method of immunological measurement of protein S which can measure free protein S at a higher sensitivity.

## SUMMARY OF THE INVENTION

Accordingly, the objects of the present invention are to eliminate the above-mentioned problems of the prior art and to provide a measurement method, measurement reagent, and kit capable of measuring free human protein S in a solution, easily and at high sensitivity. The present inventors found that free human protein S can be easily measured at a high specificity and high sensitivity by combining a monoclonal antibody which does not recognize the complex of C4bp and protein S but can specifically recognize free human protein S to be bound thereto with a polyclonal antibody which specifically recognizes human protein S and has a high affinity for an antigen.

Other objects and advantages of the present invention will be apparent from the following description.

In accordance with the present invention, there is provided a method of immunological measurement of human protein S, which comprises performing an immunological measurement of human protein S by using an antibody bound to an insoluble carrier and a labelled antibody, wherein a polyclonal antibody which specifically recognizes human protein S is used as either one of the antibodies, and a monoclonal antibody which does not recognize the complex of C4bp of the human complement system control factor and protein S but can specifically recognize free human protein S to be bound thereto is used as the other antibody.

In accordance with the present invention, there is also provided a measurement reagent for an immunological measurement of human proteins S, which comprises an antibody bound to an insoluble carrier and a labelled antibody, wherein either one of the antibodies is a polyclonal antibody which specifically recognizes human protein S, and the other antibody is a monoclonal antibody which does not recognize the complex of C4bp of the human complement system control factor with protein S but can specifically recognize free human protein S to be bound thereto.

In accordance with the present invention, there is further provided a kit for an immunological measurement of human protein S, comprising a measurement reagent for an immonological measurement of human protein S, which comprises an antibody bound to an insoluble carrier and a labelled antibody, wherein either one of the antibodies is a polyclonal antibody which specifically recognizes human protein S, and the other antibody is a monoclonal antibody which does not recognize the complex of C4bp of the human complement system control factor with protein S but can specifically recognize free human protein S to be bound thereto, which is combined with (a) a dissolving agent, (b) a washing agent, and when using an antibody labelled with an enzyme, (c) a substrate for measuring enzyme activity and a reaction stopping agent, therefor.

BRIEF DESCRIPTION OF THE DRAWINGS

The present invention will be better understood from the description set forth below with reference to the accompanying drawings wherein:

Figure 1 shows a calibration curve for a measurement of human protein S in the measurement method of the present invention (Example 1);

Fig. 2 shows a calibration curve for a measurement of human protein S in the measurement method of the present invention (Example 2);

Fig. 3 shows the results of a measurement in the co-presence of the C4bp-protein S-complex in the measurement of human protein S in the measurement method of the present invention (Example 4). In the Figure, -o- represents a human protein S concentration of 8.0 µg/ml in the sample, and -o- represents a human protein S concentration of 4.0 µg/ml in the sample; and

Fig. 4 shows a calibration curve for a measurement of human protein S in the measurement method in which a mouse anti-human protein S monoclonal antibody is used instead of the polyclonal antibody of the present invention (Comparative Example 1).

DESCRIPTION OF THE PREFERRED EMBODIMENTS

Generally, the method of measuring the presence of an amount of antigen by using an antibody which is bound to different sites of the antigen is called the sandwich method, and is described in, for example, Wide, Radioimmunoassay Methods, 199 - 206 (1970).

In the method of immunological measurement of human protein S of the present invention, as the two kinds of antibodies binding to two different sites of the antigen, a monoclonal antibody and a polyclonal antibody capable of specifically recognizing human protein S are used. As the monoclonal antibody, a monoclonal antibody which does not recognize the complex of human C4b binding protein (C4bp) which is one of the human complement system control factors and human protein S, but can specifically recognize free human protein S to be bound thereto, and as the polyclonal antibody, an antibody component of anti-human protein S antiserum which specifically recognize human protein S to be bound thereto, is used.

In the following, the method of immunological measurement of human protein S, the measurement method, and the kit used therefor according to the present invention are described in detail.

Method of immunological measurement of human protein S

A polyclonal antibody to human protein S (first antibody) is immobilized on an insoluble carrier (e.g. plastic vessel) hereinafter called "immobilized antibody"). Subsequently, to avoid nonspecific binding of the insoluble carrier with the reagent or test sample to be measured, the surface of the insoluble carrier is coated with a suitable substance (e.g. bovine serum albumin).

The thus obtained insoluble carrier having the first antibody immobilized thereon, and a test sample, are placed in contact with each other for a predetermined time and at a predetermined temperature to carry out the reaction. During this procedure, the immobilized antibody (first antibody) and human protein S in the test sample are bound together. Subsequently, after washing with an appropriate washing solution, a solution (e.g. aqueous solution) of a monoclonal antibody (second antibody) labelled with a suitable labelling substance (e.g. enzyme) is placed in contact with the human protein S bound to the immobilized antibody on the insoluble carrier for a predetermined time and at a predetermined temperature to effect the reaction with the second antibody. The product is then washed with a suitable washing solution, and the amount of labelling substance labelled on the second antibody existing on the insoluble carrier is measured.

The above reaction can be also carried out by mixing the immobilized antibody, the labelled antibody, and the test sample containing human protein S at the same time, and placing all three in contact with each other at the same time for a predetermined time and at a predetermined temperature.

Thus, the amount of free human protein S in the test sample can be calculated from the value thereof.

Constitution of measurement reagent and kit

The measurement reagent for an immunological measurement of human protein S consists of an antibody bound to an insoluble carrier and a labelled antibody as described above.

On the other hand, the kit for an immunological measurement of human protein S comprises the above measurement reagent together with the auxiliary agents, for an efficient and simple utilization of these measurement reagents, including, for example, a dissolving agent for dissolving the solid reagent or the liquid test sample, a washing agent for washing the antigen and/or antibody nonspecifically bound to the insoluble carrier, and when using an antibody labelled with enzyme, a substrate for measuring enzyme activity and a reaction stopping agent therefor, and other agents conventionally used as the kit for immunological measurement.

Examples of the insoluble carrier to be used in the method of immunological measurement of human protein S, of the present invention may include polymers such as polystyrene, polyethylene, polypropylene, polyester, polyacrylonitrile, fluorine resin, crosslinked dextran, polysaccharide, etc., or glass, metal, agarose, and combinations thereof.

The insoluble carrier can be formed in various shapes, such as a tray, sphere, fiber, rod, plate, vessel, cell, or test tube.

As the labelling substance of the labelled antibody, it is advantageous to use a radioactive substance, an enzyme, or a fluorescent substance. As the radioactive substance, $^{125}I$, $^{131}I$, $^{14}C$, $^{3}H$, can be used, and as the enzyme alkaline, phosphatase, peroxidase, β-D-galactosidase, and as the fluorescent substance, fluorescein thiocyanate, tetramethylrhodamine isothiocyanate, can be used, but these are not limitative and other elements usable in the immunological measurement method can be used.

The polyclonal antibody in the present invention may include that obtained as the antibody component of the anti-human protein S antiserum obtained by immunizing an animal with human protein S as the antigen by methods known in the art. For example, a goat anti-human protein S-polyclonal antibody or rabbit anti-human protein S-polyclonal antibody are preferably employed.

The monoclonal antibody to be used in the present invention and the method of preparation thereof are described in detail in the patent application specification filed previously as Japanese Unexamined Patent Publication No. 63-148994.

According to the present invention, free human protein S in a solution (e.g., in plasma) can be measured easily at a high specificity and high sensitivity without influence by intervening matter.

Also, according to the present invention, there are provided a reagent and a kit not existing in the prior art, which can accurately and rapidly measure free human protein S even in the co-presence of the complex of C4bp and protein S.

EXAMPLES

The present invention will now be further illustrated by, but is by no means limited to, the following Examples, wherein "%" is % by weight unless otherwise specified.

Reference Example 1: Preparation and purification of anti-human protein S (PS) monoclonal antibody

Two female Balb/C mice (4 weeks old) were immunized with purified human PS four times at intervals of 14 days. The initial immunization was effected by mixing 50 μg of human PS dissolved in PBS with an equal amount of complete Freund's adjuvant and intraperitoneally administering the resultant emulsion (0.5 mg/head), and the 2nd and 3rd immunizations by similarly mixing 50 μg of human PS with Freund's incomplete adjuvant and intraperitoneally administering same. The final immunization was effected by boosting 30 μg of human PS in a PBS solution as such through the mice tail vein. Three days after the final immunization, the spleen cells of the immunized mice were used for cell fusion.

The spleen cells of the immunized mice and myeloma cells of the same strain mice (P3U1) were mixed at a ratio of about 2:1 to about 15:1, and cell fusion was effected by the method of Kohler and Milstein, using 50% polyethylene glycyl 1540 (produced by Wako Junyaku) as the fusion accelerator. The cells after fusion were suspended in 10%FCS--RPMI-1640 medium to a cell concentration of $1 \times 10^6$ cells/ml and apportioned into a 96-wells microplate (Coster) at 100 μl per well.

The fused cell was cultured in a $CO_2$ incubator (5%, $CO_2$, 37°C), exchanged with a medium containing hypoxanthine, aminopterin, thymidine (HAT medium), grown in HAT medium, and hydridomas comprising spleen cell and myeloma cell were screened.

The antibody in the culture supernatant of hybridoma was detected by the ELISA method, using a microtiter plate coated with the antigen human PS. For the second antibody, the rabbit anti-human IgG antibody labelled with alkaline phosphatase was employed, and the binding capacity thereof to the antigen PS was examined. A colony formation was recognized in 487 wells of the 494 wells as the total of the wells seeded with the fusion cell, and 94 antibody-producing positive wells thereamong exhibited a binding capacity to the antigen PS.

Of these antibody-producing positive wells, 4 wells were subjected to cloning by the limiting dilution method, repeated twice to obtain 6 clones. The obtained clones were suspended in 90% FCS-10% DMSO and stored in liquid nitrogen.

The monoclonal antibody produced by each clone was grown intraperitoneally in Balb/C mice and the ascites therefrom were purified by using a protein A-Sepharose 4B column.

## Table 1

(Cell fusion)

| | Cell fusion-1 | Cell fusion-2 | Total |
|---|---|---|---|
| Spleen cell [cell/ml] | $1.15 \times 10^7$ | $6.70 \times 10^6$ | |
| Myeloma cell [cells/ml] | $1.72 \times 10^6$ | $1.33 \times 10^6$ | |
| Spleen cell/ myeloma cell ratio | 6.6 | 5.0 | |
| Cell number [cells/well] | $0.57 \times 10^5$ | $0.67 \times 10^5$ | |
| Well number | 302 | 192 | 494 |
| Colony formation positive well | 298 (98.6%) | 189 (98.4%) | 487 (98.6%) |
| Antibody-producing positive well exhibiting binding capacity to Protein S | 66 (21.8%) | 28 (14.8%) | 94 (19.0%) |

Reference Example 2: Properties of the purified monoclonal antibody

To determine the IgG of each clone purified from mouse ascite, the class and binding capacity thereof to human protein S were examined.

The class of mouse monoclonal antibody was determined by the Ouchterlony method, using anti-mouse antiserum specific to each class.

The results are shown in Table 2.

The binding capacity to human protein S was evaluated by reacting the monoclonal antibody diluted to an appropriate concentration with human protein S made to form a solid phase in a microtiter plate, and detecting same with goat anti-mouse IgG labelled with alkali phosphatase.

As a result, it was found that the strength of binding of 6 kinds of monoclonal antibodies to human protein S was in the order of $2B9F12 \cong 2B9C10 > 3C3G8 > 3C4G4 > 2B9G3 \gg 2E\text{-}12C7$.

## Table 2

(Class of monoclonal antibody)

| Name of antibody | Class |
|---|---|
| 2B9F12 | $IgG_1$ |
| 2B9C10 | $IgG_1$ |
| 3C3G8 | $IgG_3$ |
| 3C4G4 | $IgG_1$ |
| 2B9G3 | $IgG_1$ |
| 2E12C7 | $IgG_1$ |

Reference Example 3: Reactivity with human C4b and protein S complex

The above 6 kinds of purified monoclonal antibodies were each coated onto a microtiter plate at a concentration of 10 μg/ml, and after blocking with 1% BSA, human plasma of a healthy person diluted to an appropriate concentration was added to carry out the reaction between the C4bp-protein S complex in plasma with the monoclonal antibody. Next, an anti-C4bp antibody labelled with alkali phosphatase was added, and the binding capacity of the 6 kinds of monoclonal antibodies to the C4bp-protein S complex was detected and examined.

As a result, it was found that the monoclonal antibody 2E12C7 has a very weak binding capacity to free protein S, but exhibits a highly specific binding capacity to the C4bp-proteins S complex, with the strength of binding to the 6 kinds of monoclonal antibodies to the C4bp-protein S being in the order of $2E12C7 \gg 2B9F10 \cong 2B9C12 > 3C3G8 > 3C4G4 > 2B9G3$.

As shown in Reference Examples 2 and 3, as the monoclonal antibody which does not recognize the complex of C4bp and the protein S, but can recognize free human protein S to be bound thereto, 2B9F12 and 2B9C10 were obtained.

Example 1

(1) Preparation of antibody-immobilized beads

Beads made of polystyrene (diameter 6 mm) were left to stand in a 0.01 M phosphate buffer physiological saline (PBS) solution having a pH of 7.4 and a concentration of 20 μg/ml of a goat anti-human protein S antibody (polyclonal antibody: produced by American Diagnostica Co.) at a temperature of 4°C overnight, then washed with PBS, and thereafter, left to stand in a 0.5% aqueous bovine serum albumin (BSA) solution at a temperature of 4°C overnight for the post-coating treatment, thus obtaining antibody-immobilized beads.

(2) Preparation of monoclonal antibody labelled with horseradish peroxidase

Into 1.0 ml of a PBS solution containing 1.0 mg/ml of the monoclonal antibody (2B9F12) specifically recognizing free human protein S, 50 μl of dimethylformamide solution containing 10 mg/ml of N-(m-maleimidebenzoic acid)-N-succinimide ester (MBS) was added, and the reaction was carried out at a temperature of 25°C for 30 minutes. Then, the product was subjected to gel filtration with 0.1 M phosphate buffer (pH 6.0) by using a column filled with Sephadex G-25 to separate the maleimidated monoclonal antibody from the unreacted MBS.

On the other hand, into 2.0 ml of a PBS solution containing 1.0 mg/ml of horseradish peroxidase (HRP), 10 mg/ml of ethanolic solution of N-succinimidyl-3-(2-pyridylthiopropionate (SPDP) was added to carry out the reaction at 25°C for 30 minutes. Then, the product was purified by gel filtration with 0.01 M acetate buffer (pH 4.5) by using a column filled with Sephadex G-25, and the fractions containing the pyridyldisulfidated HRP were collected and concentrated about 10-fold in a collodion bag under ice-cooling. Next, 1 ml of 0.1 M acetate buffer (pH 4.5) containing 0.85% NaCl and 0.1 M dithiothreitol was added thereto, and the mixture was stirred at 25°C for 30 minutes to reduce the pyridyldisulfide groups introduced into the HRP molecules, followed by gel filtration with 0.1 M phosphate buffer (pH 6.0) by using a Sephadex G-25 column to obtain fractions containing thiolated HRP.

Next, the maleimidated monoclonal antibody and the thiolated HRP obtained were mixed, concentrated to a protein concentration of 4 mg/ml under ice-cooling by using a collodion bag, and left to stand at 4°C overnight, followed by gel filtration with PBS by using a column filled with ULTROgel ACA44 (produced by LKB Co., France) to obtain a HRP-labelled monoclonal antibody.

(3) Measurement of human protein S

200 µl of a PBS solution (pH 7.4) containing 0.1% BSA each containing one bead having the goat anti-human protein S antibody immobilized thereon and purified protein S at respective concentrations of 0, 50, 100, 200, 400 ng/ml, 200 µl of a PBS solution (pH 7.4) containing 0.1% BSA containing the HRP-labelled monoclonal antibody were added into each test tube (n=2) and incubated at a temperature of 37°C for one hour.

Next, after the solution in the test tube was removed by aspiration, the residue was washed twice with PBS and then 400 µl each of a 0.1 M phosphate-citrate buffer (pH 4.0) containing 0.02% of tetramethylbenzidine hydrochloride and 0.005% of hydrogen peroxide was added into each test tube to carry out incubation at a temperature of 37°C for 30 minutes, followed by enzymatic reaction with an addition of 1 ml of an aqueous solution containing 0.1% NaF and 2% acetic acid as the reaction stopping agent.

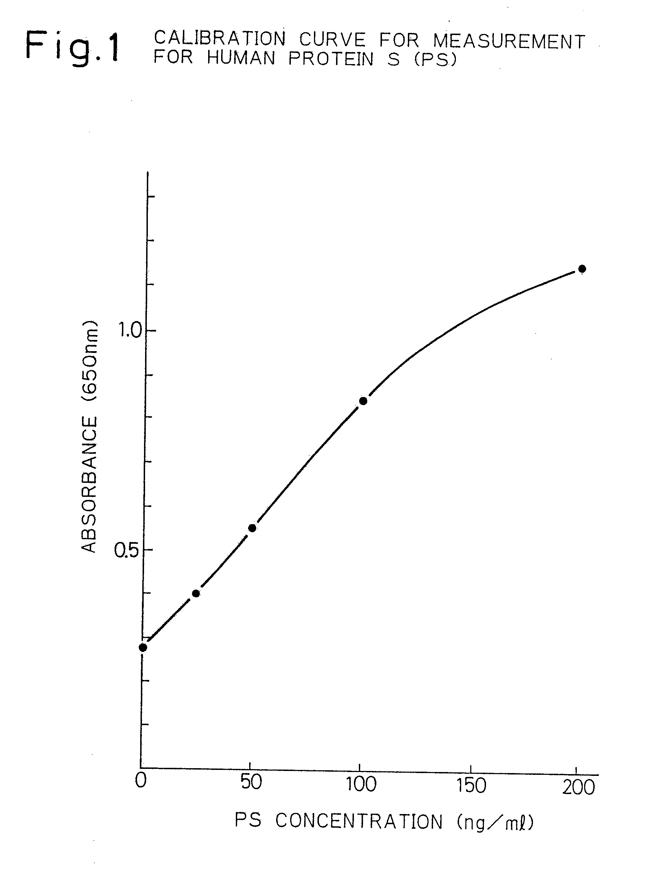

Next, the absorption strength of the solution was measured at a wavelength of 650 nm by using a spectrophotometer, and the measured values were plotted versus the human protein S concentration to obtain a calibration curve for a measurement of a human protein S concentration having a concentration dependency (see Fig. 1).

It is clear from the calibration curve shown in Fig. 1 that the calibration curve can be used for a measurement of a human protein S concentration due to its concentration depending although the base line is slightly high.

Example 2

By using the antibody-immobilized beads and the monoclonal antibody labelled with horseradish peroxidase in the same manner as in Example 1, the measurements of human protein S were carried out as follows.

200 µl of a PBS solution (pH 7.4) containing 0.1% BAS and 0.2% skimmed milk, each containing one bead having the goat anti-human protein S antibody immobilized thereon and purified protein S at respective concentrations of 0, 50, 100, 200, 400 ng/ml, 200 µl of a PBS solution (pH 7.4) containing 0.1 BSA and 0.2 % skimmed milk containing the HRP-labelled monoclonal antibody were added into each test tube (n=2) and incubated at a temperature of 37°C for one hour.

Next, after the solution in the test tube was removed by aspiration, the residue was washed twice with PBS and then 400 µl each of a 0.1 M phosphate-citrate buffer (pH 4.0) containing 0.02% of tetramethylbenzidine hydrochloride and 0.005% of hydrogen peroxide was added into each test tube to carry out incubation at a temperature of 37°C for 30 minutes, followed by enzymatic reaction with an addition of 1 ml of an aqueous solution containing 0.1% NaF and 2% acetic acid as the reaction stopping agent.

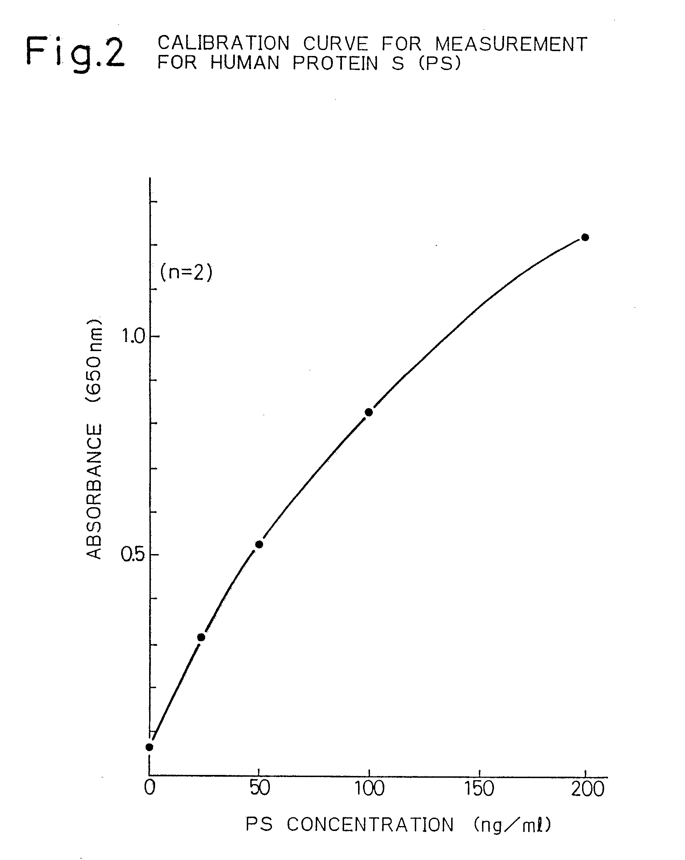

Next, the absorption strength of the solution was measured at a wavelength of 650 nm by using a spectrophotometer, and the measured values were plotted versus the human protein S concentration to obtain a calibration curve for a measurement of a human protein S concentration having a concentration dependency (see Fig. 2).

As the concentration measurement of human protein S in the plasma test sample, 200 µl of a solution of normal mixed human plasma diluted 50-fold with a PBS solution (pH 7.4) containing 0.1% BSA, and 0.2% skimmed milk was added together with the antibody-immobilized bead and 200 µl of the HRP-labelled monoclonal antibody solution into a test tube, and the immunoreaction and the chromogeneic reaction were carried out as described above, followed by a measurement of the absorbance by a spectrophotometer. As a result of the determination of the human protein S concentration calculated on the concentration in plasma by using the calibration curve in Fig. 2, the concentration in the plasma was found to be 10.4 µg/ml.

Example 3

200 µl of a PBS solution (pH 7.4) containing 0.5% BSA, each containing one bead having rabbit anti-human protein S immobilized thereon prepared by the same method as in Example 1, except for using a rabbit anti-human protein S antibody (polyclonal antibody: produced by Diagnostica Stago Co.) in place of the goat anti-human protein S antibody and purified human protein S at the respective concentration of 0, 50, 100, 200, and 400 ng/ml, and 200 µl of a PBS solution (pH 7.4) containing 0.5% BSA, containing the HRP-labelled mouse anti-human protein S monoclonal antibody prepared according to the same method as in Example 1 were added into each test tube (n = 2), followed by incubation at a temperature of 37°C for one hour.

Next, after removal of the solution in each test tube by aspiration, each residue was washed twice with PBS, and 400 μl of each of a 0.1 M phosphate-citrate buffer (pH 4.0) containing 0.02% of tetramethylbenzidine hydrochloride and 0.005% of hydrogen peroxide was added into each test tube to carry out incubation at a temperature of 37°C for 30 minutes, followed by stopping the enzymatic reaction with an addition of 1 ml of an aqueous solution containing 0.1% NaF and 2% acetic acid as the reaction stopping agent.

Next, the absorption strength of the solution was measured at a wavelength of 650 nm by using a spectrophotometer, and the measured values were plotted versus the human protein S concentration, whereby a calibration curve with a good concentration dependency was obtained, although the absorption strength of the baseline was slightly high, at 0.15.

Example 4: Measurement of human protein S in the co-presence of C4bp-protein S complex

When diluting the samples containing human protein S at the respective concentrations of 4.0 and 8.0 μg/ml with a PBS solution (pH 7.4) containing 0.1% BSA and 0.2% skimmed milk to 50-fold, the C4bp-protein S complex separated by the method of Dahlback [Dahlback, B., Seminars in Thrombosis & Hemostasis; 10; 139 - 148 (1984)] was added to the respective concentrations before dilution of 60, 120, 240, 360 and 480 μg/ml.

Next, using the respective solutions as the sample, the concentrations of human protein S were measured by the same method as in Example 2.

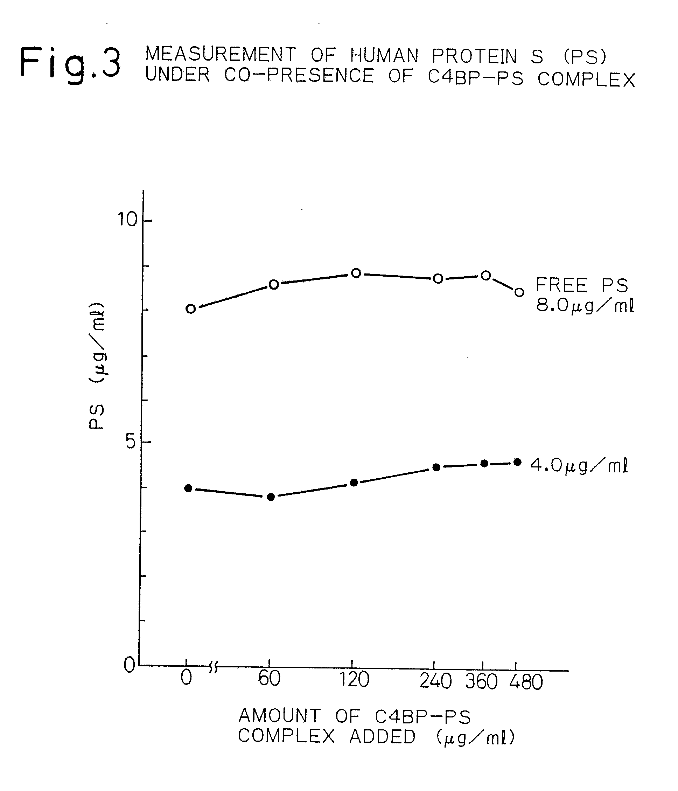

As a result, the concentration of human protein S in the sample was found to be identical with that when no C4bp-protein S complex is present within the measurement error, and thus it was shown that the present measurement method selectively measured only free human protein S without measuring the C4bp-protein S complex (see Fig. 3).

Comparative Example 1

In the preparation of the antibody-immobilized beads in this Comparative Example 1, beads were prepared by the same method as in Example 1 except for using a mouse anti-human protein S-monoclonal antibody (2B9C10) in place of the goat anti-human protein S-polyclonal antiboby. 200 μl of a PBS solution (pH 7.4) containing 0.1% BSA and 0.2% skimmed milk, containing each one bead having the mouse anti-human protein S-monoclonal antibody immobilized thereon and purified human protein S at the respective concentrations of 0, 50, 100, 200 and 400 ng/ml, and 200 μl of a PBS solution (pH 7.4) containing 0.1% BSA and 0.2% skimmed milk, containing the HRP-labelled mouse anti-human protein S-monoclonal antibody prepared by the same method as in Example 1, were added into each test tube (n = 2), followed by incubation at a temperature of 37°C for one hour.

Next, after the solution in each test tube was removed by aspiration, each residue was washed twice with PBS, and then 400 μl of each of a 0.1 M phosphate-citrate buffer (pH 4.0) containing 0.02% of tetramethylbenzidine hydrochloride and 0.005% of hydrogen peroxide was added into each test tube to carry out incubation at a temperature of 37°C for 30 minutes, followed by stopping the enzymatic reaction with an addition of 1 ml of an aqueous solution containing 0.1% NaF and 2% acetic acid as the stopping agent.

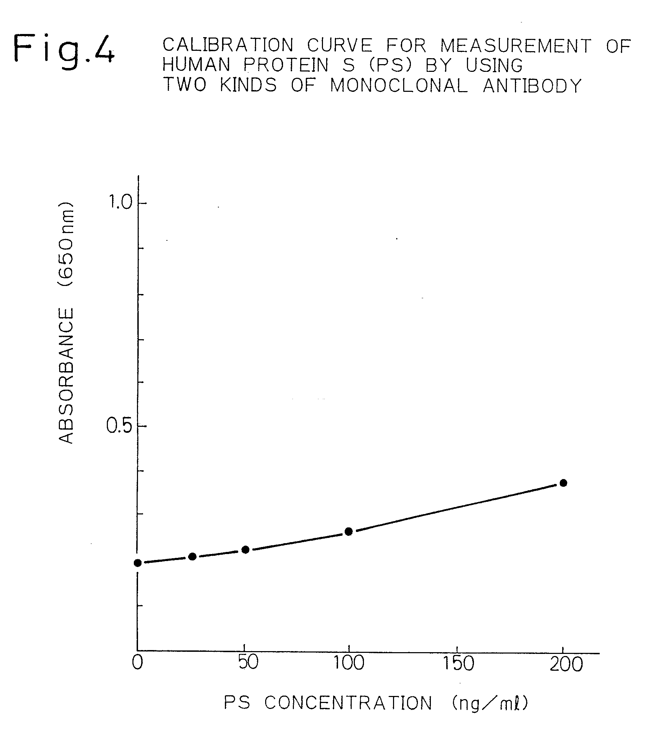

Next, the absorption strength of the solution was measured at the wavelength of 650 nm by using a spectrophotometer, and the measured values were plotted versus the human protein S concentration to obtain a calibration curve for a measurement of protein S (see Fig. 4).

As is apparent from a comparison with Fig. 1 of the present invention, the calibration curve has a baseline with a high absorbance of 0.2, and the absorbance at the protein S concentration of 200 ng/ml shows a relatively lower value of 0.38. Since the gradient of the calibration curve is very gentle, the measurement sensitivity for protein S measurement is unsatisfactory, and therefore, this system is not preferable as a measurement system. This may be considered to be because the antigen affinity of the monoclonal antibody for the protein S is not sufficiently high.

## Claims

1. A method of immunological measurement of human protein S, which comprises performing an immunological measurement of human protein S by using an antibody bound to an insoluble carrier and a labelled antibody, wherein a polyclonal antibody which specifically recognizes human protein S is used as either one of the antibodies, and a monoclonal antibody which does not recognize the complex of C4bp of a human complement system control factor and protein S but can specifically recognize free human protein S to be bound thereto, is used as the other antibody.

2. A method of immunological measurement of human protein S as claimed in claim 1, wherein the antibody bound to the insoluble carrier is a goat and/or rabbit anti-human protein S-polyclonal antibody and the labelled antibody is a mouse anti-human protein S-monoclonal antibody.

3. A measurement reagent for an immunological measurement of human protein S, which comprises an antibody bound to an insoluble carrier and a labelled antibody, wherein either one of the antibodies is a polyclonal antibody which specifically recognizes human protein S, and the other antibody is a monoclonal antibody which does not recognize the complex of C4bp of human complement system control factor and protein S but can specifically recognize free human protein S to be bound thereto.

4. A kit for an immunological measurement of human protein S, comprising a measurement reagent for immunological measurement of human protein S which comprises an antibody bound to an insoluble carrier and a labelled antibody, wherein either one of the antibodies is a polyclonal antibody which specifically recognizes human protein S, and the other antibody is a monoclonal antibody which does not recognize the complex of C4bp of a human complement system control factor with protein S but can specifically recognize free human protein S to be bound thereto, which is combined and (a) a dissolving agent (b) a washing agent and, when using an antibody labelled with an enzyme, (c) a substrate for measuring enzyme activity and a reaction stopping agent therefor.

Fig.1  CALIBRATION CURVE FOR MEASUREMENT FOR HUMAN PROTEIN S (PS)

Fig.2 CALIBRATION CURVE FOR MEASUREMENT FOR HUMAN PROTEIN S (PS)

Fig.3 MEASUREMENT OF HUMAN PROTEIN S (PS) UNDER CO−PRESENCE OF C4BP−PS COMPLEX

Fig.4    CALIBRATION CURVE FOR MEASUREMENT OF
         HUMAN PROTEIN S (PS) BY USING
         TWO KINDS OF MONOCLONAL ANTIBODY